# EUROPEAN PATENT SPECIFICATION

(11) **EP 1 881 780 B1**
(45) Date of publication and mention of the grant of the patent: **26.10.2016**
(21) Application number: 06759776.5
(22) Date of filing: 11.05.2006
(51) Int. Cl.: A61B 1/00, A61K 8/81, A61Q 1/04, A61Q 1/10

(54) **LONG WEARING GLOSSY COSMETIC COMPOSITION**
LANGANHALTENDE GLÄNZENDE KOSMETISCHE ZUSAMMENSETZUNG
COMPOSITION COSMETIQUE BRILLANTE LONGUE TENUE

(30) Priority: 12.05.2005 US 127938
(43) Date of publication of application: 30.01.2008
(73) Proprietor: ELC Management LLC, New York, NY 10153 (US)
(72) Inventor: LUO, Dexin, Fresh Meadows, NY 11365 (US); WANG, Tian, Dix Hills, New York 11746 (US); MCKENNA, Linda, North Bablyon, New York 11703 (US); MU, Weilin, Albertson, New York 11507 (US); STEPNIEWSKI, George, Melville, New York 11747 (US); NAZAR, Shahan, Garden City, New York 11530 (US)
(74) Representative: Hirsch & Associés
(86) International application number: PCT/US2006/018604
(87) International publication number: WO 2006/124706

(56) References cited:
- FR-A1- 2 783 162
- FR-A1- 2 845 278
- US-A- 4 980 155
- US-A- 4 988 502
- US-A- 5 925 337
- US-A- 6 139 827
- US-A1- 2004 126 346
- US-B1- 6 326 013

## Description

### Field of the Invention

The present invention relates to cosmetic compositions for application to the skin, including the lips and the eyelids, and to the eyelashes. The present invention also relates to cosmetic compositions, which, when dried, after application to the skin or eyelashes, exhibit excellent water- and oil-resistance and long-wear properties, and which do not readily transfer to clothing or to other surfaces. The invention also relates to cosmetic compositions that contain substantially no oils, and yet demonstrate high gloss and color intensity. The invention further relates to aqueous cosmetic compositions that do not require emulsifiers or surfactants which are typically needed to stabilize emulsion formulations.

### Background of the Invention

Vivid color, shine and long wear are desirable properties for makeup products, particularly for eyeliner and lipgloss.

Eyeliner is a particularly desirable beautifying product. It is preferred that eyeliner be intensely colored and shiny. To achieve an intensely colored black eyeliner, for example, many prior art compositions use carbon black. However, the use of carbon black results in a cosmetic product with a less than desirable level of gloss. The use of black iron oxide in other typical eyeliner formulations containing acrylates polymers and/or copolymers has resulted in products that lacked a desirable level of color intensity, since the polymers, in most cases, are white and opaque. When the polymers dry, the film is matte or exhibits a metallic or plastic shine, which generally is not desired by consumers.

However, by using water-soluble plasticizers in combination with water-soluble polymers, a transparent matrix or base is obtained that, after drying, forms a film with an oil-like shine. The high gloss intensifies the hue of colorant-containing compositions, providing a rich, deep color.

A long-wearing product that resists smudging, running and/or or fading upon exposure to oils and perspiration or tears also would be appreciated by consumers. The present invention provides transfer-resistant and water- and oil-resistant film that is long-wearing.

Non-transfer acrylates polymer-containing cosmetic compositions typically have been provided in the form of emulsions containing oils, surfactants, and/or emulsifiers or anhydrous formulations containing volatile components such as oils or alcohols. Nevertheless, products that do not contain emulsifiers, surfactants, oils and alcohols which may irritate or dry the skin, including the lips, of the user have become more desirable.

Moreover, surfactants and/or wetting agents typically have been used in cosmetic compositions for dispersing solid pigments. Such agents tend to absorb moisture because of their chemical natures. Thus the integrity of the film formed after the composition has been applied and has dried may be compromised. In the compositions of the subject invention, however, the pigments, particularly metallic oxides, are readily dispersible in the aqueous system without dispersing agents or surfactants due to the unique combination of water-soluble plasticizers and acrylates copolymers. The pigments interacting with the water-soluble components form ultrafine particles which increase the smoothness of the film formed. An eyeliner with as high coverage as a composition using carbon black is provided, but with a dewy appearance which is much preferred by consumers. The water- and oil-resistance properties of the compositions enhance their transfer-resistance.

The lipliners and lipglosses prepared according to the invention are shiny and brilliantly colored. Moreover, the water- and oil-resistance and transfer-resistance properties of the lipliner products produced enable the user to enlarge or reduce the appearance of the size of the lips which is also highly desired by consumers.

### SUMMARY OF THE INVENTION

The invention is as defined in the claims. The invention relates to cosmetic compositions containing acrylates copolymers which are truly water-soluble yet surprisingly demonstrate water-resistance and transfer-resistance upon drying on the skin, including the lips and the eyelids, and on the eyelashes. The compositions of the invention contain specific water-soluble film forming acrylic acid- or methacrylic acid-containing acrylates copolymer and a specific water-soluble plasticizer in an aqueous medium. The invention compositions contain substantially no oils and yet exhibit high gloss and color intensity. The aqueous compositions of the invention do not require surfactants and emulsifiers which are used to maintain the stability of two-phase formulations. The compositions of the invention also are water- and/or oil-resistant.

The invention also is concerned with a method of improving the transfer-resistance, the shine and/or the color intensity of a cosmetic composition by combining a specific water-soluble film-forming acrylic acid- or methacrylic acid-containing acrylates copolymer and a specific water-soluble plasticizer in an aqueous medium in the substantial absence of oils.

The invention also concerns a method for redefining the lipline which comprises providing a liplining composition comprising a transfer-resistant single phase aqueous composition comprising a specific water-soluble film forming acrylic acid- or methacrylic acid-containing acrylates copolymer and a specific water-soluble plasticizer and a pigment, preferably wherein the composition is a natural lip color composition, and tracing the composition, for example with a liplining brush, just outside or just inside the lipline, allowing the product to dry, and applying within the traced line a desired shade. Optionally, a lipgloss, comprising a transfer-resistant single phase aqueous composition comprising a water-soluble film forming acrylic acid- or methacrylic acid-containing acrylates copolymer, a water-soluble plasticizer and optionally a pigment, may be applied over the desired shade.

### DETAILED DESCRIPTION OF THE INVENTION

The novel cosmetic compositions of the invention according to claim 1 are single phase aqueous formulations containing a water-soluble film-forming acrylates copolymer, a water-soluble plasticizer, and optionally a pigment. By "single phase" it is intended that the composition is in a stable homogeneous form rather than in the form of a heterogeneous water-in-oil or oil-in-water emulsion. The film-forming acrylates copolymer and plasticizer are solublized in that single phase. Pigments, if any are present, are dispersed throughout the liquid in sufficiently small particles to remain stable in the composition. The copolymer, once dried, is neither water- nor oil-soluble. The unique combination of a film-forming water-soluble acrylates copolymer and a water-soluble plasticizer in an aqueous medium provides a product which, upon drying on the skin, including the lips, demonstrates high color intensity, high gloss and excellent long-wearing, transfer-resistant properties.

The compositions of the invention contain substantially no (less than 0.2 weight percent, if present) hydrophobic oils, and do not require the surfactants and emulsifiers which are employed to stabilize two-phase (emulsion) compositions. Typical hydrophobic oils include those disclosed, for example, in U.S. Patent Nos. 5,843,407 and 6,780,422, and in the International Cosmetic Ingredient Dictionary and Handbook, twelfth edition, 2004. Oils are those materials which are organic substances that are liquid at ambient temperature. They are esters, triglycerides, hydrocarbons and silicones.

Emulsifiers and surfactants are typically required for use in cosmetic emulsions, which contain normally immiscible water and oil phases, to disperse one of the water and the oil phases in the other phase. Typical emulsifiers and surfactants are disclosed in McCutcheon's, Vol. 1: Emulsifiers and Detergents, North American Edition, 2004. When emulsifiers and/or surfactants are present in cosmetic compositions and applied, for example, to the eyelid, the cosmetic film formed when the composition has dried still can bind water (e.g. perspiration) and oil (skin oils), thereby becoming solubilized, resulting in smudging and reduced length of wear. In contrast to emulsion compositions, the compositions of the invention do not contain hydrophobic oils; and, therefore, require no emulsifiers and surfactants to stabilize the formulations. The single phase, aqueous compositions of the invention contain truly water-soluble, film-forming copolymers which, when applied to the skin of the eyelid, for example, are not easily solublized, after drying, by either water or oil, and therefore are long-wearing, smudge-resistant and transfer-resistant. If present in the aqueous compositions of the invention, any material which demonstrates emulsifier or surfactant properties will have an HLB of less than 12.

Preferably, the water-soluble acrylates copolymer is present in the composition in an amount in the range of about 1 to about 95 percent, more preferably, in an amount of about 5 to about 70 percent, and even more preferably, in an amount of about 10 to about 30 percent, by weight of the total composition.

The water-soluble plasticizer preferably is present in the composition in an amount in the range of about 1 to about 60 percent, and more preferably, in an amount of about 2 to about 20 percent, by weight of the total composition.

The ratio of the acrylates copolymer to the water-soluble plasticizer in the invention compositions is greater than about 1:1, preferably about 2:1 to 20:1, and more preferably about 2:1 to 4:1.

The pigment, if present in the invention composition, is preferably present in an amount in the range of about 1 to about 20 percent, and more preferably is present in the amount of about 5 to about 15 percent, based on the total weight of the composition.

The water-soluble acrylates copolymer includes ethyl acrylate/methyl methacrylate/methacrylic acid; ethyl acrylate/methyl methacrylate/acrylic acid; ethyl acrylate/ethyl methacrylate/methacrylic acid; ethyl acrylate/ethyl methacrylate/acrylic acid; methyl acrylate/methyl methacrylate/methacrylic acid; methyl acrylate/methyl methacrylate/acrylic acid; methyl acrylate/ethyl methacrylate/methacrylic acid; and methyl acrylate/ethyl methacrylate/acrylic acid, particularly ethyl acrylate/methyl methacrylate/methacrylic acid and ethyl acrylate/methyl methacrylate/acrylic acid. Most preferred are Covacryl A15® and Covacryl E14® (LCW, France) which are ethyl acrylate/methylmethacrylate/methacrylic acid copolymers.

The water-soluble plasticizer is polyglyceryl-3 laurate, PEG-90 diisostearate, PEG/PPG-8/3 laurate, PEG/PPG-8/3 diisostearate, and/or triisostearoyl polyglyceryl-3 dimer dilinoleate. Most preferably, the water-soluble ester is Hydramol PGPD®, Hydramol PGPL®, Hydramol PGDS®, Hydramol TGL® or Schercemol PTID® (Industrial West, NJ). While not wishing to be bound by any theory, it is believed that the plasticizer acts like a wetting agent, which physically bonds to (coats) the pigment particles, improving pigment dispersion and contributing to the shine of the composition film when dried. Although a wetting property may be attributable to the plasticizer useful in the compositions of the invention, it is not intended that such compounds fall with the scope of the typical emulsifiers and surfactants used in conventional emulsion compositions, such emulsifiers and surfactants being substantially absent from the compositions of the invention. In fact, the plasticizers used in the present compositions could not be used in an emulsion composition as the major emulsifying agent.

The invention compositions optionally include a pigment selected from cosmetically acceptable inorganic and organic pigments, such as those disclosed in the International Cosmetic Ingredient Dictionary and Handbook, twelfth edition, 2004. The inorganic pigments may include red, black, green and yellow iron oxides, titanium dioxide, bismuth oxychloride and the like. The organic pigments may include D&C and FD&C colors. Pigments suitable for use in the invention compositions include those disclosed in U.S. Patent No. 6,726,900. Particularly preferred organic pigments are red, green, blue, yellow, violet, orange, lakes thereof and mixtures thereof, including FD&C colors Blue 1, Blue 2, Green 3, Orange B, Citrus Red 2, Red 3, Red 4, Red 40, Yellow 5, Yellow 6, Blue 1, Blue 2; Orange B, Citrus Red 2; and the D&C colors Blue 4, Blue 9, Green 5, Green 6, Green 8, Orange 4, Orange 5, Orange 10, Orange 11, Red 6, Red 7, Red 17, Red 21, Red 22, Red 27, Red 28, Red 30, Red 31, Red 33, Red 34, Red 36, Red 39, Violet 2, Yellow 7, Yellow 8, Yellow 10, Yellow 11, Blue 4, Blue 6, Green 5, Green 6, Green 8, Orange 4, Orange 5, Orange 10, Orange 11, and so on. Particularly preferred lakes are formed by the reaction of the organic pigment with a metallic salt such as aluminum, calcium, zirconium, barium, and the like. Suitable reds include pigments from the monoazo, disazo, fluoran, xanthene, or indigoid families or lakes thereof, such as Red 4, 6, 7, 17, 21, 22, 27, 28, 30, 31, 33, 34, 36, and Red 40. Also suitable are lakes of such red pigments. Typically, the metal salts are aluminum, barium, and the like. The yellow pigment may be a pyrazole, monoazo, fluoran, xanthene, quinoline, or salt thereof. Suitable yellows include Yellow 5, 6, 7, 8, 10, and 11, as well as lakes of such yellow pigments. Suitable violets include those from the anthroquinone family, such as Violet 2 and lakes thereof. Examples of orange pigments are Orange 4, 5, 10, 11, or lakes thereof.

The compositions may include further components, such as one or more water-dispersible plasticizers, for example, water-dispersible esters, such as Covaplast® (LCW, France), antimicrobials, preservatives, stabilizers, suspending agents or thickeners, water-soluble actives and combinations thereof.

Typical preservatives which may be used in the invention compositions include, for example, ethylhexylglycerin and caprylyl glycol/phenoxyethanol/hexylene glycol. Other preservatives suitable for use in the compositions are disclosed in the International Cosmetic Ingredient Dictionary and Handbook, twelfth edition, 2004.

Suitable stabilizers, suspending agents or thickeners for use in the invention compositions include, but are not limited to PVP copolymers, such as ammonium acrylodimethyltaurate/VP polymer; cellulose derivatives, for example, hydroxyethylcellulose and sodium carboxymethylcellulose; acrylates, such as glyceryl polyacrylate; polysaccharide derivatives, for example, sclerotium gel; modified corn starch; silicates, such as magnesium aluminum silicate and sodium magnesium silicate; hectorite and derivatives, such as Bentones; and xanthan gum. Additional stabilizers, suspending agents and thickeners are disclosed in McCutcheon's Volume 2: Functional Materials, North American Edition, 2004.

Water soluble actives which may be used in the compositions of the invention include sunscreens (such as Eusolex 232); and antioxidants, for example ginkgo-biloba, beta carotene, green tea, ascorbic acid and derivatives thereof such as sodium ascorbyl phosphate and magnesium ascorbyl phosphate and camosic acid (rosemary).

The compositions of the invention may be embodied in, for example, eyeliner, mascara, concealer, lip gloss and lip liner. The following non-limiting examples further illustrate the embodiments of the invention.

### EXAMPLES

### Example 1 - Eyeliner Formulation

| **TABLE 1** | | |
|---|---|---|
| **MATERIAL** | | **WEIGHT PERCENT** |
| sequence 1 | | |
| purified water | | 20.00 |
| magnesium aluminum silicate | | 0.50 |
| cellulose gum | | 0.20 |
| xanthan gum | | 0.20 |
| ethylhexylglycerin | | 0.25 |
| | | |
| sequence 2 | | |
| purified water | | 6.75 |
| ethylhexylglycerin | | 0.25 |
| iron oxides | | 13.00 |
| | | |
| sequence 3 | | |
| acrylates copolymer (Covacryl A15) | | 9.80 |
| acrylates copolymer (Covacryl E14) | | 4.00 |
| caprylylglycol/phenoxyethanol/hexylene glycol | | 0.13 |
| ethylhexylglycerin | | 0.25 |
| | | |
| sequence 4 | | |
| PEG-90 diisostearate (Hydramol PGDS) | | 2.70 |
| PEG/PPG-8/3 laurate (Hydramol PGPL) | | 1.28 |
| caprylylglycol/phenoxyethanol/hexylene glycol | | 0.15 |
| ethylhexylglycerin | | 0.25 |
| | | |
| sequence 5 | | |
| kaolin | | 5.00 |
| | | |
| sequence 6 | | |
| purified water | | 35.30 |
| | **TOTAL** | 100.00 |

With reference to Table 1, pure water and ethylhexylglycerin are heated to 50°C. Magnesium aluminum silicate, cellulose gum and xanthan gum are sprinkled into the water and ethylhexylglycerin mixture separately and stirred until the mixture is gelled. The temperature of the mixture is reduced to 25°C. forming sequence 1. Sequence 2 materials are milled together and added to the sequence 1 mixture. The materials of sequence 3 are added to the above mixture. The materials of sequences 4, 5 and 6 then are sequentially added to the above mixture. The product prepared is intensely black and shiny and transfer-resistant.

In examples 2 through 5 (Tables 2 through 5), the materials of sequence 1 are combined and mixed until uniform, the materials of sequence 2 are milled together and added to the sequence 1 mixture. The materials of each additional sequence are added to the above mixture and mixed together until uniform. The eyeliner formulations of the invention are given below in Tables 2 through 5.

### Example 2 - Eyeliner Formulation

| **TABLE 2** | | |
|---|---|---|
| **MATERIAL** | | **WEIGHT PERCENT** |
| sequence 1 | | |
| acrylates copolymer (Covacryl A15) | | 9.80 |
| acrylates copolymer (Covacryl E14) | | 4.00 |
| caprylyl glycol/phenoxyethano/hexylene glycol | | 0.25 |
| ethylhexylglycerin | | 0.50 |
| | | |
| sequence 2 | | |
| purified water | | 6.75 |
| ethylhexylglycerin | | 0.25 |
| iron oxides (cosmetic black) | | 13.00 |
| | | |
| sequence 3 | | |
| PEG-90 diisostearate (Hydramol PGDS) | | 6.00 |
| PEG/PPG-8/3 laurate (Hydramol PGPL) | | 6.00 |
| caprylylglycol/phenoxyehtanol/hexylene glycol | | 0.25 |
| ethylhexylglycerin | | 0.25 |
| | | |
| sequence 4 | | |
| purified water | | 52.95 |
| | **TOTAL** | 100.00 |

### Example 3 - Eyeliner Formulation

| **TABLE 3** | | |
|---|---|---|
| **MATERIAL** | | **WEIGHT PERCENT** |
| sequence 1 | | |
| acrylates copolymer (Covacryl E14) | | 15.00 |
| | | |
| sequence 2 | | |
| purified water | | 36.50 |
| iron oxides (cosmetic black) | | 10.00 |
| | | |
| sequence 3 | | |
| kaolin | | 30.00 |
| | | |
| sequence 4 | | |
| PEG-90 diisostearate (Hydramol PGDS) | | 0.50 |
| PEG/PPG-8/3 laurate (Hydramol PGPL) | | 8.00 |
| | **TOTAL** | 100.00 |

### Example 4 - Eyeliner Formulation

| **TABLE 4** | | |
|---|---|---|
| | | |
| **MATERIAL** | | **WEIGHT PERCENT** |
| sequence 1 | | |
| acrylates copolymer (Covacryl A15) | | 15.00 |
| acetyl tributyl citrate, tr trioctyltrimellitate, | | |
| triethyl citrate | | 0.50 |
| | | |
| sequence 2 | | |
| iron oxides | | 12.50 |
| purified water | | 7.50 |
| | | |
| sequence 3 | | |
| PEG-90 diisostearate (Hydramol PGDS) | | 2.70 |
| PEG/PPG-8/3 laurate (Hydramol PGPL) | | 1.28 |
| | | |
| sequence 4 | | |
| kaolin | | 6.00 |
| | | |
| sequence 5 | | |
| purified water | | 54.52 |
| | | |
| | **TOTAL** | 100.00 |

### Example 5 - Eyeliner Formulation

| **TABLE 5** | | |
|---|---|---|
| **MATERIAL** | | **WEIGHT PERCENT** |
| sequence 1 | | |
| acrylates copolymer (Covacryl A15) | | 9.80 |
| acrylates copolymer (Covacryl E14) | | 4.00 |
| | | |
| sequence 2 | | |
| iron oxides | | 12.50 |
| purified water | | 7.50 |
| | | |
| sequence 3 | | |
| PEG-90 diisostearate (Hydramol PGDS) | | 3.00 |
| | | |
| sequence 4 | | |
| kaolin | | 6.00 |
| | | |
| sequence 5 | | |
| purified water | | 57.20 |
| | | |
| | **TOTAL** | 100.00 |

### Example 6 - Lipgloss Formulation

| **TABLE 6** | | |
|---|---|---|
| **MATERIALS** | | **WEIGHT PERCENT** |
| sequence 1 | | |
| acrylates copolymer (Covacryl A15) | | 7.68 |
| acrylates copolymer (Covacryl E14) | | 3.42 |
| purified water | | 35.90 |
| | | |
| sequence 2 | | |
| purified water | | 10.00 |
| titanium dioxide | | 1.42 |
| iron oxides (pure oxy red 78054/3080) | | 0.63 |
| D&C red no. 7 calcium lake (C19-011) | | 0.60 |
| iron oxides (cosmetic yellow C-33-8073/CG490) | | 0.34 |
| iron oxides (pure oxy black 7053/3068) | | 0.25 |
| | | |
| sequence 3 | | |
| mica/titanium dioxide (Timiron MP-1005 supersilk) | | 1.00 |
| | | |
| sequence 4 | | |
| PEG-90 diisostearate (Hydramol PGDS) | | 3.00 |
| PEG/PPG-8/3 laurate (Hydramol PGPL) | | 3.00 |
| | | |
| sequence 5 | | |
| purified water | | 30.00 |
| hectorite (Bentone EW) | | 0.60 |
| caprylyl glycol/phenoxyethanol/hexylene glycol | | 0.50 |
| ethylhexylglycerin | | 0.50 |
| | | |
| sequence 6 | | |
| purified water | | 1.16 |
| | **TOTAL** | 100.00 |

With reference to Table 6, sequence 1 materials are mixed until uniform. Sequence 2 materials are milled together until uniform and added to the sequence 1 mixture. Sequence 3 and 4 materials are sequentially added to the above mixture, mixing until uniform. Sequence 5 materials are premixed, heated to 75°C. until uniform, and added to the above mixture. The mixture is then mixed at room temperature until uniform. The product prepared is glossy, vivid in color, transfer-resistant, and comfortable on the lips.

### Example 7 - Lipgloss Formulation

| **TABLE 7** | | |
|---|---|---|
| **MATERIALS** | | **WEIGHT PERCENT** |
| sequence 1 | | |
| acrylates copolymer (Covacryl A15) | | 7.68 |
| acrylates copolymer (Covacryl E14) | | 3.42 |
| purified water | | 35.90 |
| | | |
| sequence 2 | | |
| PEG-90 diisostearate (Hydramol PGDS) | | 3.00 |
| PEG/PPG-8/3 laurate (Hydramol PGPL) | | 3.00 |
| | | |
| sequence 3 | | |
| purified water | | 30.00 |
| caprylyl glycol/phenoxyethanol/hexylene glycol | | 0.50 |
| ethylhexylglycerin | | 0.50 |
| | | |
| sequence 4 | | |
| purified water | | 16.00 |
| | **TOTAL** | 100.00 |

The composition of Example 7 is prepared in the same manner as the composition of Example 6 except that the latter is an unpigmented lipgloss.

### Example 8 - Comparative Gloss test

| **TABLE 8** | | |
|---|---|---|
| **MATERIALS** | | **WEIGHT PERCENT** |
| sequence 1 | | |
| acrylates copolymer (Covacryl | | |
| A15) | | 9.80 |
| acrylates copolymer (Covacryl | | |
| E14) | | 4.00 |
| caprlyl glycol/phenoxyethanol/hexylene glycol | | 0.15 |
| purified water | | 32.10 |
| | | |
| sequence 2 | | |
| purified water | | 23.94 |
| caprlyl glycol/phenoxyethanol/hexylene glycol | | 0.15 |
| Ethylhexylglycerin | | 0.13 |
| iron oxides (cosmetic black) | | 12.75 |
| | | |
| sequence 3 | | |
| kaolin | | 6.30 |
| caprlyl glycol/phenoxyethanol/hexylene glycol | | 0.20 |
| | | |
| sequence 4 | | |
| PEG-90 diisostearate (Hydramol PGDS) | | 2.70 |
| PEG/PPS-8/3 laurate (Hydramol PGPL) | | 1.28 |
| purified water | | 6.30 |
| caprlyl glycol/phenoxyethanol/hexylene glycol | | 0.20 |
| | **TOTAL** | 100.00 |

An eyeliner formulation having the components shown in Table 8 is prepared by mixing sequence 1 materials together at room temperature until uniform, milling together sequence 2 materials, adding the milled sequence 2 materials to the sequence 1 mixture and mixing until uniform. Sequence 3 materials are added to the mixture of sequence 1 and 2 materials, and followed by the addition of the sequence 4 materials and mixing until uniform. Test samples (5 mm wet thickness) of the formulation of the invention (sample A) and three comparative eyeliner product emulsion formulations (samples B, C and D) are cast on Leneth card (form 2A-Opacity) within a 1" X 2" area. The samples are sufficiently thick (opaque) to avoid background reflectance. The samples are permitted to dry. Light is flashed at the samples at angles of 20° and 60°. The reflectance (scale of 0 - 100 gloss units) for each sample is measured at angles of 20° and 60°, using a gloss meter from BYK Garden; Model: micro-TRI-gloss dried. As indicated in Table 9 below, the inventive composition (A) demonstrated a higher gloss than any of the comparative emulsion compositions (B-D).

| **TABLE 9** | | |
|---|---|---|
| SAMPLE | GLOSS UNITS | |
| | 20° | 60° |
| A (inv) | 0.5 | 5.4 |
| B (comp) | 0.2 | 2.1 |
| C (comp) | 0.1 | 0.4 |
| D (comp) | 0.1 | 1.7 |

Note: Comparative sample B contains greater than 0.2 weight percent oil (Carnauba wax). Comparative sample C contains greater than 0.2 weight percent oil (neopentyl glycol/C13-C14 isoparaffin). Comparative sample C contains greater than 0.2 weight percent oil (oleyl alcohol).

### Example 9 - Evaluation of water-based eyeliner for transfer-resistance

A study is done to determine the resistance of the eyeliner formulation shown in Table 8 to wear and flaking. Eight adult women participate in the study. The volunteer panelists are in normal health with no evidence of a systemic illness, nor any dermatogical disorder in the areas used in the study, which conditions might interfere with the analysis of the test results. Pregnant or lactating volunteers are excluded from the study. Panelists used for the study are not using systemic or topical retinoids, antihistamines or similar agents during the course of the study and two weeks prior to commencement of the study.

The women are instructed to wear no moisturizer or makeup for the test. The test sites are the eyelids. The panelists are given a sample of liquid eyeliner and instructed to apply the eyeliner as evenly as possible to the top and the bottom eyelids.

Wear and flaking evaluations are carried out immediately after product application, and two, four, six and eight hours after application.

Wear and flaking are assessed via photography using a Fuji S2 digital camera. The panelist's head is fixed in a headrest (Canfield Scientific) to which the camera is mounted. The camera lens is set at a distance of 0.35m from the area to be photographed. Close up photographs of the eye area are taken at two, four, six and eight hours after application. The photographs are stored and viewed on Canfield Photofile Image Management Software (version 4.5.148). Clinical evaluations of the photographs are conducted by a trained investigator using a 10-point analog scale (shown below). The investigator is trained and qualified by an outside consultant to objectively identify and quantify the characteristics of skin parameters. The investigator has an extensive perceptual vocabulary, and is experienced in scale usage and the use of standardized evaluation techniques. A standard lexicon and references specifically for each of wear and flaking parameters (i.e. a photo scale depicting what a "0" looks lie, what a "2' looks like, etc. up to "10") are used for evaluation. "Wear" is defined as the visual observation of the amount of product color appearing and the amount of natural skin color that is visible at the indicated time points after application. "Flaking" is defined as the visual observation of the number of pieces of product falling onto the skin around the eyes or into the eyes at the indicated time points after application.

| 10 point scale | | |
|---|---|---|
| 10 | | |
| No wear | | Extreme wear |
| No flaking | | Extreme flaking |

The results of the clinical evaluation are shown in Table 10 below. After 8 hours of wear, the eyeliner composition of the invention demonstrated minimal wear and minimal flaking.

| **TABLE 10** | | | | |
|---|---|---|---|---|
| Time After Application | Observed Wear | | Observed Flaking | |
| 0 | 0.0 | (none) | 0.0 | (none) |
| 2 | 0.0 | (none) | 0.0 | (none) |
| 4 | 0.0 | (none) | 0.0 | (none) |
| 6 | 0.4 | (minimal) | 0.0 | (none) |
| 8 | 0.8 | (minimal) | 0.7 | (minimal) |

## Claims

1. A transfer-resistant, single phase aqueous cosmetic composition comprising one or more of each of:
- a film-forming water-soluble acrylates copolymer selected from the group consisting of ethyl acrylate/methyl methacrylate/methacrylic acid; ethyl acrylate/methyl methacrylate/acrylic acid; ethyl acrylate/ethyl methacrylate/methacrylic acid; ethyl acrylate/ethyl methacrylate/acrylic acid; methyl acrylate/methyl methacrylate/methacrylic acid; methyl acrylate/methyl methacrylate/acrylic acid; methyl acrylate/ethyl methacrylate/methacrylic acid; and methyl acrylate/ethyl methacrylate/acrylic acid, and
- a water-soluble ester selected from the group consisting of polyglyceryl-3 laurate, PEG-90 diisostearate, PEG/PPG - 8/3 laurate, PEG/PPG -8/3 diisostearate, triisostearoyl polyglyceryl-3 dimer dilinoleate, or a mixture thereof;
wherein the composition contains substantially no oils.

2. The composition according to claim 1, further comprising a pigment.

3. The cosmetic composition according to claim 1 or 2, wherein the water-soluble acrylates copolymer is present in an amount of from about 5 to about 70 percent by weight of the total composition.

4. The cosmetic composition according to claim 1 or 2, wherein the water-soluble ester is present in an amount of from about 2 to about 20 percent by weight of the total composition.

5. The cosmetic composition according to claim 2, wherein the pigment is selected from the group consisting of cosmetically acceptable inorganic and organic pigments.

6. The cosmetic composition according to claim 5, wherein the pigment is present in an amount of from about 1 to about 20 percent by weight of the total composition.

7. The cosmetic composition according to claim 2, which is an eyeliner, a mascara, a concealer, a lipgloss or a lipliner.

8. The transfer-resistant, single phase aqueous cosmetic composition according to claim 2, comprising from about 10 to about 30 weight percent ethyl acrylate/methylmethacrylate/methacrylic acid copolymer, from about 2 to about 20 weight percent PEG-90 diisostearate and PEG/PPG -8/3-laurate, and from about 5 to about 15 weight percent black iron oxide, based on the total weight of the composition.

9. The transfer-resistant, single phase aqueous cosmetic composition according to claim 2, comprising from about 10 to about 30 weight percent ethyl acrylate/methylmethacrylate/methacrylic acid copolymer, from about 2 to about 20 weight percent PEG-90 diisostearate, and from about 5 to about 15 weight percent black iron oxide, based on the total weight of the composition.

10. The transfer-resistant, single phase aqueous cosmetic composition according to claim 1, comprising from about 10 to about 30 weight percent ethyl acrylate/methylmethacrylate/methacrylic acid copolymer, and from about 2 to about 20 weight percent PEG-90 diisostearate and PEG/PPG -8/3-laurate.

11. The transfer-resistant, single phase aqueous cosmetic composition, comprising from about 10 to about 30 weight percent ethyl acrylate/methylmethacrylate/methacrylic acid copolymer, and from about 2 to about 20 weight percent PEG-90 diisostearate.

12. A method of improving the transfer-resistance and/or the shine of a cosmetic composition according to claim 1, comprising combining:
water;
one or more water-soluble-film-forming acrylates copolymers selected from the group consisting of ethyl acrylate/methyl methacrylate/methacrylic acid; ethyl acrylate/methyl methacrylate/acrylic acid; ethyl acrylate/ethyl methacrylate/methacrylic acid; ethyl acrylate/ethyl methacrylate/acrylic acid; methyl acrylate/methyl methacrylate/methacrylic acid; methyl acrylate/methyl methacrylate/acrylic acid; methyl acrylate/ethyl methacrylate/methacrylic acid; and methyl acrylate/ethyl methacrylate/acrylic acid; and
one or more water-soluble esters selected from the group consisting of polyglyceryl-3 laurate, PEG-90 diisostearate, PEG/PPG - 8/3 laurate, PEG/PPG -8/3 diisostearate, triisostearoyl polyglyceryl-3 dimer dilinoleate, or a mixture thereof;
wherein the composition contains substantially no oil.

13. A method of improving the transfer-resistance and/or the shine and/or the color intensity of a cosmetic composition according to claim 2, comprising combining:
water;
one or more water-soluble film-forming acrylates copolymers selected from the group consisting of ethyl acrylate/methyl methacrylate/methacrylic acid; ethyl acrylate/methyl methacrylate/acrylic acid; ethyl acrylate/ethyl methacrylate/methacrylic acid; ethyl acrylate/ethyl methacrylate/acrylic acid; methyl acrylate/methyl methacrylate/methacrylic acid; methyl acrylate/methyl methacrylate/acrylic acid; methyl acrylate/ethyl methacrylate/methacrylic acid; and methyl acrylate/ethyl methacrylate/acrylic acid; and
one or more water-soluble esters selected from the group consisting of polyglyceryl-3 laurate, PEG-90 diisostearate, PEG/PPG - 8/3 laurate, PEG/PPG -8/3 diisostearate, triisostearoyl polyglyeryl-3 dimer dilinoleate, or a mixture thereof; and
pigment;
wherein the composition contains substantially no oil.

14. A method of redefining the lipline, comprising:
providing a first and a second transfer-resistant single phase aqueous composition according to claim 2, wherein the pigment of the first composition has a natural lip color, and the pigment of the second composition has a second desired color;
dipping a lipliner brush into the first composition having the first natural lip color so as to load the brush with the composition;
tracing a line with the lipliner brush just inside or just outside a user's natural lipline;
allowing the traced line to dry;
dipping a lipliner brush into the second composition having the second desired lip color so as to load the brush with the composition; and
applying the second desired color composition within the redefined lipline.

15. The method according to claim 14, which further comprises applying over the second composition having the second desired lip color a lipgloss composition comprising a transfer-resistant single phase aqueous composition according to claim 1.

16. The method according to claim 15, wherein the lipgloss composition further comprises a pigment.

## Patentansprüche

1. Übertragungsresistente, wässrige, kosmetische Einphasenzusammensetzung umfassend eines oder mehrere von jedem von:
- einem filmbildenden, wasserlöslichen Acrylatcopolymer, ausgewählt aus der Gruppe bestehend aus:
Ethylacrylat/Methylmethacrylat/Methacrylsäure;
Ethylacrylat/Methylmethacrylat/Acrylsäure;
Ethylacrylat/Ethylmethacrylat/Methacrylsäure;
Ethylacrylat/Ethylmethacrylat/Acrylsäure;
Methylacrylat/Methylacrylat/Methacrylsäure;
Methylacrylat/Methylmethacrylat/Acrylsäure;
Methylacrylat/Ethylmethacrylat/Methacrylsäure;
und
Methylacrylat/Ethylmethacrylat/Acrylsäure, und
- einem wasserlöslichen Ester ausgewählt aus der Gruppe bestehend aus Polyglyceryl-3-laurat, PEG-90-diisostearat, PEG/PPG-8/3-laurat, PEG/PPG-8/3-diisostearat, Triisostearoylpolyglyceryl-3-dimer-dilinoleat oder einer Mischung davon;
wobei die Zusammensetzung im Wesentlichen keine Öle enthält.

2. Zusammensetzung nach Anspruch 1, ferner ein Pigment umfassend.

3. Kosmetische Zusammensetzung nach Anspruch 1 oder 2, wobei das wasserlösliche Acrylatcopolymer in einer Menge von etwa 5 bis etwa 70 Gewichtsprozent der gesamten Zusammensetzung vorliegt.

4. Kosmetische Zusammensetzung nach Anspruch 1 oder 2, wobei der wasserlösliche Ester in einer Menge von etwa 2 bis etwa 20 Gewichtsprozent der gesamten Zusammensetzung vorliegt.

5. Kosmetische Zusammensetzung nach Anspruch 2, wobei das Pigment aus der Gruppe ausgewählt ist bestehend aus kosmetisch akzeptablen anorganischen und organischen Pigmenten.

6. Kosmetische Zusammensetzung nach Anspruch 5, wobei das Pigment in einer Menge von etwa 1 bis etwa 20 Gewichtsprozent der gesamten Zusammensetzung vorliegt

7. Kosmetische Zusammensetzung nach Anspruch 2, die ein Lidstrich, eine Wimperntusche, ein Concealer, ein Lipgloss oder ein Lippenkonturenstift ist.

8. Übertragungsresistente, wässrige, kosmetische Einphasenzusammensetzung nach Anspruch 2, umfassend etwa 10 bis etwa 30 Gewichtsprozent Ethylacrylat/Methylmethacrylat/Methacrylsäure-Copolymer, etwa 2 bis etwa 20 Gewichtsprozent PEG-90-diisostearat und PEG/PPG-8/3-laurat und etwa 5 bis etwa 15 Gewichtsprozent schwarzes Eisenoxid, auf das Gesamtgewicht der Zusammensetzung bezogen.

9. Übertragungsresistente, wässrige, kosmetische Einphasenzusammensetzung nach Anspruch 2, umfassend etwa 10 etwa 30 Gewichtsprozent Ethylacrylat/Methylmethacrylat/Methacrylsäure-Copolymer, etwa 2 bis etwa 20 Gewichtsprozent PEG-90-diisostearat und etwa 5 bis etwa 15 Gewichtsprozent schwarzes Eisenoxid, auf das Gesamtgewicht der Zusammensetzung bezogen.

10. Übertragungsresistente, wässrige, kosmetische Einphasenzusammensetzung nach Anspruch 1, umfassend etwa 10 bis etwa 30 Gewichtsprozent Ethylacrylat/Methylmethacrylat/Methacrylsäure-Copolymer und etwa 2 bis etwa 20 Gewichtsprozent PEG-90-diisostearat und PEG/PPG-8/3-laurat.

11. Übertragungsresistente, wässrige, kosmetische Einphasenzusammensetzung, umfassend etwa 10 bis etwa 30 Gewichtsprozent Ethylacrylat/Methylmethacrylat/Methacrylsäure-Copolymer und etwa 2 bis etwa 20 Gewichtsprozent PEG-90-diisostearat.

12. Verfahren zum Verbessern der Übertragungsresistenz und/oder des Glanzes einer kosmetischen Zusammensetzung nach Anspruch 1, umfassend das Kombinieren:
- von Wasser;
- von einem oder mehreren wasserlöslichen filmbildenden Acrylatcopolymeren ausgewählt aus der Gruppe bestehend aus:
Ethylacrylat/Methylmethacrylat/Methacrylsäure;
Ethylacrylat/Methylmethacrylat/Acrylsäure;
Ethylacrylat/Ethylmethacrylat/Methacrylsäure;
Ethylacrylat/Ethylmethacrylat/Acrylsäure;
Methylacrylat/Methylacrylat/Methacrylsäure;
Methylacrylat/Methylmethacrylat/Acrylsäure;
Methylacrylat/Ethylmethacrylat/Methacrylsäure; und
Methylacrylat/Ethylmethacrylat/Acrylsäure, und
- einem oder mehreren wasserlöslichen Estern ausgewählt aus der Gruppe bestehend aus Polyglyceryl-3-laurat, PEG-90-diisostearat, PEG/PPG-8/3-laurat, PEG/PPG-8/3-diisostearat, Triisostearoylpolyglyceryl-3-dimerdilinoleat oder einer Mischung davon;
wobei die Zusammensetzung im Wesentlichen kein Öl enthält.

13. Verfahren zum Verbessern der Übertragungsresistenz und/oder des Glanzes und/oder der Farbintensität einer kosmetischen Zusammensetzung nach Anspruch 2, umfassend das Kombinieren:
- von Wasser;
- einem oder mehreren wasserlöslichen filmbildenden Acrylatcopolymeren ausgewählt aus der Gruppe bestehend aus:
Ethylacrylat/Methylmethacrylat/Methacrylsäure;
Ethylacrylat/Methylmethacrylat/Acrylsäure;
Ethylacrylat/Ethylmethacrylat/Methacrylsäure;
Ethylacrylat/Ethylmethacrylat/Acrylsäure;
Methylacrylat/Methylacrylat/Methacrylsäure;
Methylacrylat/Methylmethacrylat/Acrylsäure;
Methylacrylat/Ethylmethacrylat/Methacrylsäure; und
Methylacrylat/Ethylmethacrylat/Acrylsäure; und
- einem oder mehreren wasserlöslichen Estern ausgewählt aus der Gruppe bestehend aus Polyglyceryl-3-laurat, PEG-90-diisostearat, PEG/PPG-8/3-laurat, PEG/PPG-8/3-diisostearat, Triisostearoylpolyglyceryl-3-dimerdilinoleat oder einer Mischung davon; und
- Pigment;
wobei die Zusammensetzung im Wesentlichen kein Öl enthält.

14. Verfahren zum Redefinieren der Lippenlinie, umfassend:
- das Bereitstellen einer ersten und einer zweiten übertragungsresistenten, wässrigen Einphasenzusammensetzung nach Anspruch 2, wobei das Pigment der ersten Zusammensetzung eine natürliche Lippenfarbe aufweist und das Pigment der zweiten Zusammensetzung eine zweite erwünschte Farbe aufweist;
- Tauchen des Lippenkonturpinsels in die erste Zusammensetzung, die die erste natürliche Lippenfarbe aufweist, um den Pinsel mit der Zusammensetzung zu beladen;
- Ziehen einer Linie mit dem Lippenkonturpinsel direkt innerhalb oder direkt außerhalb der natürlichen Lippenlinie eines Benutzers;
- Gestatten, dass die gezogene Linie trocken wird;
- Tauchen eines Lippenkonturpinsel in die zweite Zusammensetzung, die die zweite erwünschte Lippenfarbe aufweist, um den Pinsel mit der Zusammensetzung zu beladen; und
- Aufbringen der zweiten erwünschten Farbzusammensetzung innerhalb der redefinierten Lippenlinie.

15. Verfahren nach Anspruch 14, das ferner das Aufbringen über der zweiten Zusammensetzung, die die zweite erwünschte Lippenfarbe aufweist, einer Lipgloss-Zusammensetzung umfassend eine übertragungsresistente, wässrige Einphasenzusammensetzung nach Anspruch 1.

16. Verfahren nach Anspruch 15, wobei die Lipgloss-Zusammensetzung ein Pigment umfasst.

## Revendications

1. Composition aqueuse cosmétique monophasique, résistant au transfert, comprenant un ou plusieurs éléments parmi :
- un copolymère d'acrylates hydrosoluble filmogène, choisi dans le groupe constitué par l'acrylate d'éthyle/le méthacrylate de méthyle/l'acide méthacrylique ; l'acrylate d'éthyle/le méthacrylate de méthyle/l'acide acrylique ; l'acrylate d'éthyle/le méthacrylate d'éthyle/l'acide méthacrylique ; l'acrylate d'éthyle/le méthacrylate d'éthyle/l'acide acrylique ; l'acrylate de méthyle/le méthacrylate de méthyle/l'acide méthacrylique ; l'acrylate de méthyle/le méthacrylate de méthyle/l'acide acrylique ; l'acrylate de méthyle/le méthacrylate d'éthyle/l'acide méthacrylique ; et l'acrylate de méthyle/le méthacrylate d'éthyle/l'acide acrylique ; et
- un ester hydrosoluble choisi dans le groupe constitué par le polyglycéryl-3 laurate, le PEG-90 diisostéarate, le PEG/PPG - 8/3 laurate, le PEG/PPG-8/3 diisostéarate, le triisostéaroyl polyglycéryl-3 dimère dilinoléate ou un mélange de ceux-ci ;
dans laquelle la composition ne contient sensiblement aucune huile.

2. Composition selon la revendication 1, comprenant en outre un pigment.

3. Composition cosmétique selon la revendication 1 ou 2, dans laquelle le copolymère d'acrylates hydrosoluble est présent en une quantité allant d'environ 5 à environ 70 % en poids de la composition totale.

4. Composition cosmétique selon la revendication 1 ou 2, dans laquelle l'ester hydrosoluble est présent en une quantité allant d'environ 2 à environ 20 % en poids de la composition totale.

5. Composition cosmétique selon la revendication 2, dans laquelle le pigment est choisi dans le groupe constitué des pigments inorganiques et organiques cosmétiquement acceptables.

6. Composition cosmétique selon la revendication 5, dans laquelle le pigment est présent en une quantité allant d'environ 1 à environ 20 % en poids de la composition totale.

7. Composition cosmétique selon la revendication 2, qui est un crayon pour le contour des yeux, un mascara, un anticerne, un brillant à lèvres ou un crayon pour le contour des lèvres.

8. Composition aqueuse cosmétique monophasique résistant au transfert selon la revendication 2, comprenant d'environ 10 à environ 30 % en poids de copolymère d'acrylate d'éthyle/ de méthacrylate de méthyle/d'acide méthacrylique, d'environ 2 à environ 20 % en poids de PEG-90 diisostéarate et de PEG/PPG-8/3-laurate, et d'environ 5 à environ 15 % en poids d'oxyde de fer noir, sur la base du poids total de la composition.

9. Composition aqueuse cosmétique monophasique résistant au transfert selon la revendication 2, comprenant d'environ 10 à environ 30 % en poids de copolymère d'acrylate d'éthyle/ de méthacrylate de méthyle/d'acide méthacrylique, d'environ 2 à environ 20 % en poids de PEG-90 diisostéarate, et d'environ 5 à environ 15 % en poids d'oxyde de fer noir, sur la base du poids total de la composition.

10. Composition aqueuse cosmétique monophasique résistant au transfert selon la revendication 1, comprenant d'environ 10 à environ 30 % en poids de copolymère d'acrylate d'éthyle/ de méthacrylate de méthyle/d'acide méthacrylique, et d'environ 2 à environ 20 % en poids de PEG-90 diisostéarate et de PEG/PG-8/3-laurate.

11. Composition aqueuse cosmétique monophasique résistant au transfert, comprenant d'environ 10 à environ 30 % en poids de copolymère d'acrylate d'éthyle/ de méthacrylate de méthyle/d'acide méthacrylique et d'environ 2 à environ 20 % en poids de PEG-90 diisostéarate.

12. Procédé pour l'amélioration de la résistance au transfert et/ou de la brillance d'une composition cosmétique selon la revendication 1, consistant à combiner :
de l'eau ;
un ou plusieurs copolymères d'acrylates hydrosolubles filmogènes, choisis dans le groupe constitué par l'acrylate d'éthyle/le méthacrylate de méthyle/l'acide méthacrylique ; l'acrylate d'éthyle/le méthacrylate de méthyle/l'acide acrylique ; l'acrylate d'éthyle/le méthacrylate d'éthyle/l'acide méthacrylique ; l'acrylate d'éthyle/le méthacrylate d'éthyle/l'acide acrylique ; l'acrylate de méthyle/le méthacrylate de méthyle/l'acide méthacrylique ; l'acrylate de méthyle/le méthacrylate de méthyle/l'acide acrylique ; l'acrylate de méthyle/le méthacrylate d'éthyle/l'acide méthacrylique ; et l'acrylate de méthyle/le méthacrylate d'éthyle/l'acide acrylique ; et
un ou plusieurs esters hydrosolubles choisis dans le groupe constitué par le polyglycéryl-3 laurate, le PEG-90 diisostéarate, le PEG/PPG - 8/3 laurate, le PEG/PPG-8/3 diisostéarate, le triisostéaroyl polyglycéryl-3 dimère dilinoléate ou un mélange de ceux-ci ;
dans lequel la composition ne contient sensiblement aucune huile.

13. Procédé pour l'amélioration de la résistance au transfert et/ou de la brillance et/ou de l'intensité colorée d'une composition cosmétique selon la revendication 2, consistant à combiner :
de l'eau ;
un ou plusieurs copolymères d'acrylates hydrosolubles filmogènes, choisis dans le groupe constitué par l'acrylate d'éthyle/le méthacrylate de méthyle/l'acide méthacrylique ; l'acrylate d'éthyle/le méthacrylate de méthyle/l'acide acrylique ; l'acrylate d'éthyle/le méthacrylate d'éthyle/l'acide méthacrylique ; l'acrylate d'éthyle/le méthacrylate d'éthyle/l'acide acrylique ; l'acrylate de méthyle/le méthacrylate de méthyle/l'acide méthacrylique ; l'acrylate de méthyle/le méthacrylate de méthyle/l'acide acrylique ; l'acrylate de méthyle/le méthacrylate d'éthyle/l'acide méthacrylique ; et l'acrylate de méthyle/le méthacrylate d'éthyle/l'acide acrylique ; et
un ou plusieurs esters hydrosolubles choisis dans le groupe constitué par le polyglycéryl-3 laurate, le PEG-90 diisostéarate, le PEG/PPG - 8/3 laurate, le PEG/PPG-8/3 diisostéarate, le triisostéaroyl polyglycéryl-3 dimère dilinoléate ou un mélange de ceux-ci ; et
du pigment ;
dans lequel la composition ne contient sensiblement aucune huile.

14. Procédé pour la redéfinition du contour des lèvres, comprenant les étapes consistant à :
fournir une première et une deuxième compositions aqueuses monophasiques résistant au transfert selon la revendication 2, dans lesquelles le pigment de la première composition possède une couleur naturelle des lèvres, et le pigment de la deuxième composition possède une deuxième couleur souhaitée ;
plonger la brosse du crayon pour le contour des lèvres dans la première composition ayant la première couleur naturelle des lèvres de manière à charger la brosse avec la composition ;
tracer un trait avec la brosse du crayon pour le contour à lèvres juste à l'intérieur ou juste à l'extérieur d'un contour naturel des lèvres d'une utilisatrice ;
laisser sécher le trait tracé ;
plonger la brosse du crayon pour le contour des lèvres dans la deuxième composition ayant la deuxième couleur souhaitée des lèvres de manière à charger la brosse avec la composition ; et
appliquer la deuxième composition de couleur souhaitée à l'intérieur du trait redéfini des lèvres.

15. Procédé selon la revendication 14, qui comprend en outre l'étape consistant à appliquer sur la deuxième composition ayant la deuxième couleur souhaitée des lèvres une composition de brillant à lèvres comprenant une composition aqueuse monophasique résistant au transfert selon la revendication 1.

16. Procédé selon la revendication 15, dans lequel la composition de brillant à lèvres comprend en outre un pigment.
